# EUROPEAN PATENT APPLICATION

(11) **EP 3 424 496 A1**
(43) Date of publication of application: **09.01.2019**
(21) Application number: 17760207.5
(22) Date of filing: 17.01.2017
(51) Int. Cl.: A61K 9/16, A61K 31/4184

(54) **PREPARATION CONTAINING ESOMEPRAZOLE**

(30) Priority: 29.02.2016 KR 20160024669
(71) Applicant: Yoo Young Pharm Co., Ltd., Seoul 06687 (KR)
(72) Inventor: KIM, Jung Ju, Seoul 06093 (KR); KUK, Yun Mo, Seoul 05823 (KR); SON, Hyung Min, Seoul 02635 (KR)
(74) Representative: Louis Pöhlau Lohrentz
(86) International application number: PCT/KR2017/000550
(87) International publication number: WO 2017/150803

(57) **Abstract**

The present invention relates to a preparation containing esomeprazole as an active ingredient. The present invention is characterized in that a controlled-release system is introduced such that the efficacy of esomeprazole, which is an active ingredient, can be continuously exhibited.

## Description

### TECHNICAL FIELD

The present invention relates to the design of a drug product containing esomeprazole as an active pharmaceutical ingredient, and more specifically, to technology for a drug delivery system of an oral preparation consisting of a plurality of enteric-coated unit sections.

### BACKGROUND FIELD

The development of a drug is carried out according to the search of an active pharmaceutical ingredient (API) and the determination of a drug product. Herein, the determination of a drug product is called formulation. Formulation starts from the selection of a dosage form.

According to the classification system in the Korean Pharmacopoeia, there are 36 kinds of dosage forms; however, the dosage forms are greatly classified into tablets, capsules, injections, eye drops, external preparations or other semisolid forms. The selection of a dosage form is made based upon the preformulation study result of an API. That is, a dosage form is chosen from the dosage forms that can optimize the expression of the pharmacological effects of the API, after conducting searches on solubility, lipophilicity, pKa, stability in the solution state, penetrability, stability in the body, Pharmacokinetics (PK), etc. of the API. Of course, in the selection of the dosage form, a drug compliance of a patient or an increase of distribution convenience of a drug is also the matters to be considered.

After the selection of the dosage form, based upon the preformulation result of the API, a drug delivery system is specifically determined. For example, in case where a dosage form for oral administration, such as a preparation or a capsule, is selected, if the preformulation result shows that the API stimulates the stomach, is well absorbed in the small intestine, or is unstable in the gastric fluid, the drug delivery system is designed as delayed-release.

When the selection of the drug delivery system is finished, the prescription of the drug product is finally made by determining the ingredients and amounts of additives. As the ingredients of the additives, materials that are harmless to humans have been known in HANDBOOK OF PHARMACEUTICAL EXCIPIENTS, etc. and unless there is a special circumstance, the ingredients are selected from those materials.

The present invention relates to the design of a drug product, and basically contains esomeprazole as an active pharmaceutical ingredient, and its dosage form is a tablet or capsule, and it has a delayed-release drug delivery system. However, there are known inventions containing esomeprazole as an active pharmaceutical ingredient, wherein an enteric-coated oral tablet or capsule form is adopted.

For instance, commercially available "Nexium Tab" is an enteric-coated tablet containing esomeprazole as an active pharmaceutical ingredient. In addition, Korean Patent No. 1104349 discloses an enteric-coated tablet and capsule wherein the insufficiency of the stability and properties of the active pharmaceutical ingredient of "Nexium Tab" was improved by solid-dispersing with magnesium oxide and povidone.

The enteric-coated tablet disclosed in Korean Patent No. 1104349 will be explained. Said enteric-coated tablet is prepared through the following process. Povidone is dissolved in ethanol, a solution in which NaOH is dissolved is added therein and mixed, and then esomeprazole is added and completely dissolved, and a part of magnesium oxide is added in this solution and dispersed. This solution is sprayed to colloidal silicon dioxide and magnesium oxide in a fluidized bed to prepare a granule. This granule is mixed with microcrystalline cellulose, crospovidone and magnesium stearate and tableted to prepare a tablet. The prepared tablet is coated with HPMC using a tablet coater to make a separating layer, and is enteric-coated thereon with HPMC P(HP-50) or a methacrylic ethylacrylate copolymer (1:1).

Subsequently, the capsule disclosed in Korean Patent No. 1104349 is prepared through the following process. Povidone is dissolved in ethanol, and magnesium oxide is sprayed thereto, and then a solution in which esomeprazole is added and completely dissolved is sprayed to a spherical white sugar in the fluidized bed to prepare a pellet. This pellet is coated with HPMC, and is enteric-coated thereon with a methacrylate copolymer dispersion solution. The capsule is filled with the enteric-coated pellet.

### DETAILED DESCRIPTION OF INVENTION

### SUMMARY OF INVENTION

Since esomeprazole has acid lability, this needs to be enteric-coated. For an enteric-coated preparation, it is an essential matter to be considered that this preparation is designed such that the preparation can be rapidly disintegrated in the small intestine and its active pharmaceutical ingredient can be rapidly dissolved and absorbed. However, the enteric-coated tablet of Korea Patent No. 1104349 has a limitation in the disintegration rate because its surface area is small. In the aspect of the rapid disintegration, it is desirable to increase the surface area by making the size small; however, in the case of tablets, when considering a drug compliance and distribution convenience, there is a limitation in the reduction of the size. For this reason, it is advised that an enteric-coated preparation consists of granules or grains which have a wider surface area than a tablet.

The capsule of Korean Patent No. 1104349 consists of enteric-coated pellets. Since the pellet is a kind of granules which have a wide surface area from the viewpoint of the tablet, this may be superior to tables in the aspect of the disintegration rate. However, according to the test results of the present inventor, the pellets of Korean Patent No. 1104349 have a limitation in solubilizing the active pharmaceutical ingredient. Thus, the present inventor undertook the present invention in order to improve the solubility of the active pharmaceutical ingredient implemented with the pellets.

Meanwhile, basically, drugs that exhibit the efficacy fast after administration and at the same time, last the efficacy for a long time are better. For this, it is necessary to be designed such that the concentration is continuously maintained after the concentration in the plasma of the active pharmaceutical ingredient released from the drug product rapidly reaches the effective blood concentration. If the active pharmaceutical ingredient is early released in the excess amount, there would be no problem in rapidly reaching the effective blood concentration, but there would be a difficulty in continuously maintaining the effective blood concentration. In this aspect, Korean Patent No. 1104349 wherein in the drug delivery system, only delayed-release is introduced with consideration for the acid-lability of esomeprazole is insufficient to last the drug efficacy. Therefore, the present inventor was concerned in the present invention about the additional introduction of a release-control system that can maintain the durability of the drug release.

### MEANS FOR ACHIEVING TASK

The present invention has solved the aforementioned task through the following means.
(1) A preparation comprising a plurality of unit sections including a first section pellet comprising an inert core, a drug layer coated on the inert core, and an outermost layer coated with an enteric layer; and a second section pellet comprising an inert core, a drug layer coated on the inert core, and an outermost layer coated with an enteric layer, characterized in that the drug layer of the first section pellet contains esomeprazole as an active ingredient, and the drug layer of the second section pellet contains esomeprazole as an active ingredient and meglumine as a solubilizer, wherein in the first section, when testing with 100 rotations per minute according to the first elution test method, the active ingredient in the unit section is released in the amount of 5% or less relative to the total amount until 10 minutes after elution initiation, and the active ingredient is released in the amount of 80% or more relative to the total amount until 30 minutes, and in the second section, when testing with 100 rotations per minute according to the first elution test method, the active ingredient in the unit section is released in the amount of 5% or less relative to the total amount until 3 hours after elution initiation, and the active ingredient is released in the amount of 70% or more relative to the total amount until 5 hours.
(2) The preparation according to (1), characterized in that the first section pellet further comprises a separating layer between the drug layer and the outermost layer coated with the enteric layer.
(3) The preparation according to (1) or (2), characterized in that the second section pellet further comprises a separating layer between the drug layer and the outermost layer coated with the enteric layer, wherein the separating layer comprises a sustained release polymer.
(4) The preparation according to any one of (1) to (3), characterized in that the drug layer of the second section pellet further comprises a sustained release polymer.
(5) The preparation according to any one of (1) to (4), characterized in that the drug layer of the second section pellet further comprises a surfactant.
(6) The preparation according to (5), characterized in that the surfactant is polysorbate.

### EFFECT OF INVENTION

The present invention is excellent in the stability of the active pharmaceutical ingredient even though the present invention does not form a solid dispersion, unlike Korean Patent No. 1104349.

In addition, the present invention is excellent in the durability of the drug efficacy because the drug delivery system is designed such that the active pharmaceutical ingredient can be continuously released in the small intestine.

Further, in case of introducing a delayed-release system into an enteric-coated pellet, the absorption of the delayed-released active pharmaceutical ingredient must be possible within the limited residence time in the small intestine. In this aspect, the preset invention has no problem because the present invention is excellent in the solubilization of the active pharmaceutical ingredient in the sustained enteric-coated pellet.

Other additional effects will be explained below.

### BRIEF DESCRIPTION OF DRAWINGS

Fig. 1 shows the elution profiles of the first section pellet (F1) and the second section pellet (F2) according to the present invention when conducting the elution test according to the first elution test method. In Fig. 1, the respective points indicate values at 10 min, 30 min, 180 min, 300 min and 480 min in order.
Fig. 2 shows the comparison of the elution profiles of the capsule (F3) according to the present invention and the commercially available control preparation, when conducting the elution test according to the first elution test method.
Fig. 3 shows the comparison of the animal test (Beagle) PK profiles of the capsule (F3) according to the present invention and the commercially available control preparation.
Fig. 4 shows the comparison of the animal test (Beagle) PD profiles (gastric acid secretion inhibition rate) of the capsule (F3) according to the present invention and the commercially available control preparation.
Fig. 5 shows the comparison of the elution profiles according to the amount of meglumine used.
Figs. 6 and 7 show the evaluation of the stability of esomeprazole in the capsule (F3) according to the present invention.
Fig. 8 shows the measurement (SEM) of the surfaces of the pellet (F2) in which polysorbate 80 was used as the surfactant and the pellet (F3) in which monoglyceride was used as the surfactant. The upper figure shows the profile of F2, and the lower figure shows the profile of F7.
Fig. 9 shows the similarity between the PK profile and the elution profile of F3 in the animal test.

### BEST MODE FOR EMBODIMENT OF INVENTION

The present invention contains esomeprazole as an active pharmaceutical ingredient. Esomeprazole, which is one of omeprazole racemic mixtures, is a proton pump inhibitor. Since esomeprazole is effective in inhibiting gastric acid secretion and is useful as an antiulcer drug, this has been used for the prevention and treatment of diseases related to gastric acid.

It has been known that esomeprazole is liable to be sequentially disintegrated and transformed at an acidic or neutral pH. Further, the stability of esomeprazole is sensitively influenced by humidity and an organic solvent. For this reason, for preparations containing esomeprazole, it was focused on improving the stability of esomeprazole.

First, for esomeprazole, it is required to design the drug delivery system as delayed-release in consideration of acid-lability. Delayed-release refers to a system for inhibiting the release of a drug in the stomach and delaying the release until the drug reaches the intestine. Generally, delayed-release is introduced in case where a drug is not stable in the gastric mucosa environment; in case where a drug stimulates the gastric mucosa or causes side effects such as nausea and vomiting; or in case where a drug is specifically absorbed well in the small intestine.

Delayed-release can be implemented by enteric-coating a tablet or capsule with an enteric polymer, which inhibits or minimizes the release of a drug in the acidic environment of the stomach and releases the drug at pH of the intestine. Polymers that ionize at various pH ranges are commercially available, and depending on the ionization property of polymers, the delay degree of the release of the drug after the drug reaches the intestine can be adjusted.

When enteric-coating, greatly, the two points should be considered. First, coating should be evenly made such that the drug product is not disintegrated at an acidic or neutral pH. Generally, coating is formed by spraying a coating solution to a tablet or granule. Herein, if the coating solution is not evenly applied on the tablet or granule, a part that is insufficiently coated would protrude, so that the delayed-release of the drug product would be interrupted. Secondly, the drug product should be rapidly disintegrated when the drug reaches the absorption target gastrointestinal tract region. The residence time in each site of the gastrointestinal tract when the drug product is orally administered is 2-3 hours in the stomach, 4-6 hours in the small intestine, and 24-72 hours in the large intestine. As such, the residence time in the small intestine is longer than that in the stomach; however, it cannot be assured that this is sufficient time to remove the coating and disintegrate the drug product. For this reason, it is necessary to make the surface area of the enteric-coated tablet or granule large by reducing the size or making the shape close to the spherical shape.

Based on the aforementioned concept, the present invention containing esomeprazole as an active pharmaceutical ingredient was implemented as an enteric preparation, and particularly, the prescription was designed such that the basic unit of the preparation consists of a plurality of pellet sections that have a relatively small size and have the shape close to the spherical shape. Sometimes, in the case of implementing a pellet according to any prescription, there was also a case where the shape is not close to the spherical shape. It was confirmed, however, that surprisingly, the prescription according to the present invention makes it possible to evenly apply the enteric-coating agent and is designed such that the entire shape is close to the spherical shape.

Furthermore, the characteristic of the present invention lies in that delayed-release is introduced as the drug delivery system, unlike general enteric preparations. It is sufficient in the expression of the drug efficacy if an active pharmaceutical ingredient is absorbed in the body at above effective blood concentration. For this reason, the excess absorption according to the large early release of the active pharmaceutical ingredient is not helpful in the continuous expression of the drug efficacy. If the drug delivery system of delayed-release is introduced into a drug product, the continuous expression of the drug efficacy may be possible. However, there is no commercially available drug that contains esomeprazole as an active pharmaceutical ingredient wherein the drug delivery system of delayed-release is applied. The reason is because the attempt to combine the drug delivery systems of delayed-release and sustained release was not general and because there was a technical difficulty in implementing such system.

First, in order to delayed-release acid-labile esomeprazole, it is required to design the prescription which can ensure the stability of esomeprazole for a long time in the body. As aforementioned, considering that the residence time in the gastrointerstinal tract of a drug is 2-3 hours in the stomach and 4-6 hours in the small intestine, in order to delayed-release esomeprazole, it should be stable for at least 7 hours after oral administration. However, since the stability of esomeprazole is considerably sensitive to humidity and a solvent as well as pH, there was a technical difficulty in securing the stability of esomeprazole in the drug product remaining in the gastrointerstinal tract for a long time.

Next, in order to delayed-release esomeprazole in the small intestine, it should be solubilized such that the active pharmaceutical ingredient released from the drug product designed as delayed-release can also be absorbed in the body within 4-6 hours which are the time the drug medicine residues in the small intestine. For example, if the active pharmaceutical ingredient is released when 5 hours passed after the drug product residues in the small intestine, within the range of total 6 hours for which the drug product can residue in the small intestine, the absorption in the small intestine is possible only when the active pharmaceutical ingredient is rapidly solubilized for the remaining short time. However, in the past, the prescription study for solubilization of esomeprazole was insufficient.

However, in spite of the aforementioned technical difficulty, the present inventor designed a preparation with the system comprising a plurality of unit sections including a first section pellet comprising an inert core, a drug layer coated on the inert core, and an outermost layer coated with an enteric layer; and a second section pellet comprising an inert core, a drug layer coated on the inert core, and an outermost layer coated with an enteric layer, and at the same time implemented such that the first section is immediate-released and the second section is delayed-released, and in particular, the second section pellet designed to be delayed-released is mixed with meglumine (or meglumine and surfactant) as a solubilizer of esomeprazole in the drug layer so as to rapidly solubilize the delayed-released esomeprazole.

Hereinafter, the prescription and characteristics will be explained.

### [Definition]

In the present specification, "esomeprazole" refers to an active ingredient exhibiting pharmacological activity, and this includes all of the forms of the main ingredients such as various salts, prodrug, etc., that a person skilled in the art can combine. For example, it should be interpreted that esomeprazole magnesium trihydrate is also included in "esomeprazole."

"Pellet" refers to a medicament-containing particle having a diameter in the range of about 100-1500 microns. A pellet which has a shape close to the spherical shape is better.

"Inert core" refers to a medicament-free spherical inert material, and this is a basic seed for preparing a pellet by additionally applying an immediate-release drug layer or sustained release drug layer on its outer portion. For example, sugar sphere or spherical microcrystalline cellulose, etc. can be used.

"Surfactant" refers to a material that has hydrophilic groups and lipophilic groups in the molecule at the same time and is dissolved or dispersed in a solvent and selectively adhered to an interface so as to significantly change the property of the interface. The unlimited examples include sodium lauryl sulfate (SLS) and poloxamer, etc.

"Sustained release polymer" refers to a polymer substrate that makes an active pharmaceutical ingredient to be biologically available during the duration after the oral administration. Ingredients that can be used as a sustained release polymer have been known, and the examples include HPMC, etc.

"Enteric layer" refers to a layer coated with an enteric-coating agent that is not dissolved even in any buffered aqueous solution having about 1.0 to 8.0 of pH. Water-insoluble polymers that can be used as an enteric-coating agent have been known, and the examples include ethylcellulose or methacrylic acid acrylic acid copolymer, etc.

"First elution test method" refers to the elution test using the rotating basket method corresponding to Device 1 of the elution test method defined in the Korean Pharmacopoeia. The elution test method tests an oral preparation to determine whether the preparation is suitable for the elution test standard, and this is one of the test methods for the purpose of preventing significant biological nonequivalence. The sample of this test is equivalent to the minimum administration dose, and unless otherwise expressly provided, this means one tablet for tablets, one capsule for capsules, and the defined amount for other preparations.

The elution test methods are divided into 1) the first method (rotating basket method), 2) the second method (paddle method), and 3) the third method (Flow-Through Cell method), depending on the device used. The device of the first method (rotating basket method) consists of a container of a glass or transparent chemically inert material with a cap, a motor, a rotation axis and a cylindrical basket. The container is installed in the proper size of a constant-temperature water bath or put in a constant-temperature cover (jacket), etc. and is heated. The constant-temperature water bath or constant-temperature cover is adjusted such that when conducting the test, the temperature in the container is 37 ± 0.5 °C and also the liquid in the constant-temperature water bath smoothly moves. It should be careful not to make a shake or vibration caused by the device or the surrounding environment where the device is installed, other than the smooth rotation of the stirring unit. It should be made to observe the sample and the stirring state during the operation. The container, which is cylindrical while its lower part is hemispherical, has 1000 mL content, 160 ∼ 210 mm height, and 98 ∼ 106mm inner diameter, and an edge protrudes on the top of the container. In order to prevent the evaporation of the test solution, the container is covered with a cap. The distance of any part of the rotation axis from the center axis in the vertical direction of the container should be within 2 mm so as to rotate smoothly, so that a shake or vibration which influences the result does not occur. The number of rotations is adjusted such that it rotates within the range of ± 4 % of the defined number of rotations. The rotation axis and the basket are made with a stainless steel or an equivalent inert material. In addition, a basket coated with metal at a thickness of 2.5 µm can be used. When starting the test, the sample is put into the dried basket. During the operation, the distance between the bottom of the inner side of the container and the end of the lower side of the basket is fixed to be 25 ± 2 mm.

Meanwhile, the specific ingredients of various additives mentioned in the present specification, including "inert core", "surfactant", "sustained release polymer" and "enteric-coating agent" can be optionally selected from the pharmaceutically available materials known in the HANDBOOK OF PHARMACEUTICAL EXCIPIENTS, etc. unless there is a special limitation.

### [First section pellet]

The present invention relates to a preparation wherein the unit sections such as a plurality of pellets are assembled. Among these pellets, a first section pellet and a second section pellet are essentially comprised, wherein the first section pellet is configured to be fast release enteric, and the second section pellet is configured to be sustained release enteric.

The first section pellet comprises an inert core, a drug layer coated on the inert core, and an outermost layer coated with an enteric layer. A separating layer can be further introduced between the drug layer and the enteric layer, if necessary.

The inert core can consist of a generally recognizable size, component when preparing an enteric pellet. For instance, spherical sugar sphere having a diameter in the range of 100-1500 micron can be selected.

The drug layer coated on the inert core contains esomeprazole and a binding agent, and can be further mixed with any additive. As the binding agent, a cellulose derivative can be used, preferably, methylcellulose, hydroxypropylcellulose or hydroxypropylmethylcellulose can be used, and more preferably, hydroxypropylmethylcellulose can be used. As any additive, an excipient, a disintegrant, a lubricant or a surfactant, etc. can be mixed.

According to one embodiment of the present invention, a surfactant can be mixed with the drug layer. As the surfactant, polysorbate, sorbitan esters, poly(oxy-1,2-ethanediyl) derivative can be used, and among these, in the aspect of the expression of the effect of having a solid surface profile while the shape of the implemented pellet is closer to the spherical shape, the surfactant is preferably selected from one or more of polysorbate 20, polysorbate 40, polysorbate 60 and polysorbate 80, and more preferably, polysorbate 80 can be adopted.

Particularly, the present inventor was the first to confirm the spherical shape through the SEM (Scanning electron microscope) measurement after the preparation of the product using two kinds of surfactants whose hydrophile-lipophile balance (HLB) values are different, and as the result, it was confirmed that polysorbate having the higher HLB value (HLB ≥ 15) served as a preferable role in the formation of the spherical shape as compared to monoglyceride (HLB 3.3 ∼ 4.1) having a low HLB value.

The amounts of the respective ingredients can be properly selected by a person skilled in the art. For instance, the binding agent can be comprised in the amount of 5-25 wt.% relative to the total weight of the drug layer, and the surfactant can be comprised in the amount of 0.5-5 wt.% relative to the total weight of the drug layer.

The drug layer can be applied on the inert core according to any coating method. For instance, the drug layer can be prepared by dissolving esomeprazole and the binding agent in purified water and 70% ethanol, dispersing them with an absorbent, and then filtering it to prepare a coating solution, and then spraying the solution to the inert core.

According to one embodiment of the present invention, a separating layer can be added on the drug layer.

The separating layer comprises a binding agent and a lubricant, and can be further mixed with any additive.

As the binding agent, a cellulose derivative can be used, preferably, methylcellulose, hydroxypropylcellulose or hydroxypropylmethylcellulose can be used, and more preferably, hydroxypropylmethylcellulose can be used.

As the lubricant, metal salts (talc, magnesium stearate, calcium stearate, sodium stearylfumarate and zinc stearate) and non-metal salts (fatty acid esters, fatty acids, alcohols, fumaric acid, polyethyleneglycol, polytetrafluoroethylene lubricant) lubricants can be used, preferably, the lubricant is selected from one or more of metal salt lubricants, and more preferably, talc can be used.

The amounts of the respective ingredients can be properly selected by a person skilled in the art, and it is characterized in that preferably, the binding agent is comprised in the amount of 2-5 wt.% relative to the total weight of the separating layer comprising the drug layer, and the lubricant is comprised in the amount of 2-15 wt.% relative to the total weight of the separating layer comprising the drug layer.

The separating layer can be applied on the core of the drug layer according to any coating method. For instance, the separating layer can be prepared by stirring the binding agent and talc in purified water and dispersing it, and then filtering it to prepare a coating solution, and then spraying the solution to the core of the drug layer.

In the first section pellet, an enteric layer is applied on the outermost layer. The enteric layer can be prepared by adding a water-insoluble polymer and a plasticizer into a mixed solvent of water : ethanol = 5 : 95 and stirring and dispersing it to prepare a coating solution and then spraying the solution to the outermost layer of the core.

The enteric layer comprises a water-insoluble polymer, a lubricant and a plasticizer, and can be further mixed with any additive. As the water-insoluble polymer, a methacrylic acid ethylacrylic acid copolymer, a methacrylic acid methylmethaacrylate copolymer, a hydroxypropylmethylcellulosephthalate, hydroxypropylmethylcellulose acetate succinate, polyvinylacetylphthalate or cellulose acetatephthalate can be used, and preferably, a methacrylic acid ethylacrylic acid copolymer can be used.

As the lubricant, metal salts (talc, magnesium stearate, calcium stearate, sodium stearylfumarate and zinc stearate) and non-metal salts (fatty acid esters, fatty acids, alcohols, fumaric acid, polyethyleneglycol, polytetrafluoroethylene lubricant) lubricants can be used, and the lubricant is preferably selected from one or more of metal salt lubricants, and more preferably, talc can be used.

As the plasticizer, esters such as triethyl citrate, medium-chain fatty acid triglyceride, diethyl phthalate, dibutyl phthalate, triacetin, butyl phthalyl butyl glycolate, glyceryl caprylate ester, etc. and alcohols such as glycerine, propylenglycol, polyethylene glycol, etc. can be used, and preferably, triethyl citrate can be used.

The amounts of the respective ingredients can be properly selected by a person skilled in the art, and preferably, the water-insoluble polymer can be comprised in the amount of 10-20 wt.% relative to the total weight of the first section, the lubricant can be comprised in the amount of 5-10 wt.% relative to the total weight of the first section, and the plasticizer can be comprised in the amount of 2-5 wt.% relative to the total weight of the first section. The above amount of the water-insoluble polymer is to obtain the elution result of the first section according to the present invention, and is to meet the drug release time of Nexium which is a control preparation.

The first section pellet has the shape close to the spherical shape and has a solid surface profile.

The first section pellet designed such that when testing with 100 rotations per minute according to the first elution test method, the active ingredient in the unit section is released in the amount of 5% or less relative to the total amount until 10 minutes after elution initiation, and the active ingredient is released in the amount of 80% or more relative to the total amount until 30 minutes is suitable for achieving the effective blood concentration. Hereinafter, being suitable means that it is not that the blood concentration of the drug is too high as the active pharmaceutical ingredient is released in the excess amount, and on the contrary to this, it is not that the blood concentration of the drug cannot reach the effective blood concentration because the release amount of the active pharmaceutical ingredient is too small.

The present invention is implemented by the aforementioned prescription such that the first section pellet can meet the above elution pattern.

### [Second section pellet]

The second section pellet is unit section that delayed-releases esomeprazole as compared to the first section pellet.

The second section pellet comprises an inert core, a drug layer coated on the inert core, and an outermost layer coated with an enteric layer. A separating layer can be further introduced between the drug layer and the enteric layer, if necessary.

The inert core can be configured along the same lines as that of the first section pellet.

The drug layer coated on the inert core contains esomeprazole, meglumine and a binding agent, and can be further mixed with any additive. The binding agent and any additive can be configured along the same lines as those of the first section pellet.

Meanwhile, the drug layer of the second section pellet can be further mixed with any known sustained release polymer, unlike the drug layer of the first section pellet.

It is the main point that the drug layer of the second section is mixed with meglumine. Meglumine is a component generally known as an alkalizing agent. However, surprisingly, the present inventor has accidentally found that meglumine could improve the solubility of esomeprazole, and thus was able to have completed the present invention. Since the second section pellet is the delayed-release unit section, the release time of esomeprazole is later than that of the first section pellet. Thus, when considering the limited residence time of the drug in the small intestine, the second section pellet needs to improve the solubility of released esomeprazole. Examples of solubilizing agents of general drugs include micronisation of particles, mixing with a poloxamer, and solid dispersion formation, etc. These are all technologies applicable in the fast release drug delivery system, and known technologies for the drug delivery system in which delayed-release and controlled release are complexed were insufficient. Particularly, there has been no solubilizing means suitable for delayed-release and controlled release of esomeprazole. However, the present inventor was the first to improve the solubility of esomeprazole designed as delayed-release and controlled release by mixing with meglumine.

In addition, the drug layer of the second section can be preferably mixed with the surfactant that can be mixed in the first section. In the case of further mixing the surfactant, the solubility of esomeprazole can be improved according to the synergistic effect with meglumine, and in addition, if polysorbate 80 is selected from the surfactants, it is possible to prepare a pellet which has the shape close to the spherical shape and has a solid surface profile as explained above in the first section.

The amounts of the respective ingredients can be properly selected by a person skilled in the art, and preferably, the binding agent can be comprised in the amount of 5-25 wt.% relative to the total weight of the drug layer, the surfactant can be comprised in the amount of 0.5-5 wt.% relative to the total weight of the drug layer, and meglumine can be comprised in the amount of 25-55 wt.% relative to the total weight of the drug layer in order to obtain the target elution result of the second section.

The drug layer can be prepared along the same lines as in the first section pellet.

According to one embodiment of the present invention, a separating layer can be added on the drug layer. The prescription and preparation method of the separating layer can be made along the same lines as in the first section pellet. However, the amounts of the respective ingredients can be properly selected by a person skilled in the art, and preferably, the binding agent can be comprised in the amount of 2-5 wt.% relative to the total weight of the separating layer comprising the drug layer, and the lubricant can be comprised in the amount of 2-15 wt.% relative to the total weight of the separating layer comprising the drug layer.

In the second section pellet, an enteric layer is applied on the outermost layer. The enteric layer comprises a water-insoluble polymer, a lubricant and a plasticizer, and can be further mixed with any additive. As the water-insoluble polymer, a methacrylic acid ethylacrylic acid copolymer, a methacrylic acid methylmethaacrylate copolymer, a hydroxypropylmethyl cellulosephthalate, hydroxypropylmethylcellulose acetate succinate, polyvinylacetylphthalate and cellulose acetatephthalate can be used, and preferably, a methacrylic acid methylmethaacrylate copolymer can be used.

Herein, the reason why the water-insoluble polymer of the first section and the water-insoluble polymer of the second section are different is because of pH independency that each coating has, and in L30D55 of the first section, a coating is dissolved at above pH 5.5, and in water-insoluble polymer L100 of the second section has pH 6.0 and S100 has pH 7.0, and in order to release the drug at the desired time at pH 6.5 required in the present invention, L100 and S100 can be mixed for use.

As the lubricant, metal salts (talc, magnesium stearate, calcium stearate, sodium stearylfumarate and zinc stearate) and non-metal salts (fatty acid esters, fatty acids, alcohols, fumaric acid, polyethyleneglycol, polytetrafluoroethylene lubricant) lubricants can be used, preferably, the lubricant is selected from one or more of metal salt lubricants, and more preferably, talc can be used.

As the plasticizer, esters such as triethyl citrate, medium-chain fatty acid triglyceride, diethyl phthalate, dibutyl phthalate, triacetin, butylphthalylbutylglycolate, glycerylcaprylate ester, etc. and alcohols such as glycerine, propylenglycol, polyethylene glycol, etc. can be used, and preferably, triethyl citrate can be used.

The amounts of the respective ingredients can be properly selected by a person skilled in the art, and it is characterized in that preferably, the water-insoluble polymer can be comprised in the amount of 5-30 wt.% relative to the total weight of the second section, the lubricant can be comprised in the amount of 5-10 wt.% relative to the total weight of the second section, and the plasticizer can be comprised in the amount of 2-5 wt.% relative to the total weight of the second section.

The above amount of the water-insoluble polymer is to release the drug at a specific time in order to obtain the elution result of the second section targeted in the present invention.

The enteric layer can be prepared by putting the water-insoluble polymer and the plasticizer into a mixed solvent of water : ethanol = 5 : 95 and stirring and dispersing it to prepare a coating solution and then spraying the solution to the outermost layer of the core. The prescription is made such that the enteric layer of the second section pellet is delayed-released, unlike the enteric layer of the first section pellet.

The second section pellet has a shape close to the spherical shape and has a solid surface profile.

The second section pellet designed such that when testing with 100 rotations per minute according to the first elution test method, the active ingredient in the unit section is released in the amount of 5% or less relative to the total amount until 3 hours after elution initiation, and the active ingredient is released in the amount of 70% or more relative to the total amount until 5 hours is suitable for lasting the efficacy of esomeprazole. Herein, being suitable means that the blood concentration of the drug can be maintained at the effective blood concentration continuously.

The present invention is implemented by the aforementioned prescription such that the second section pellet can meet the above elution pattern.

### [Dosage form]

The preparation of the present invention comprises the first section pellet and the second section pellet. For instance, the capsule can be filled with the first section pellet and the second section pellet to prepare the final drug product. The amounts of the active ingredients in the first section and the second section can be designed in the proper amount by mixing them at a proper ratio by a person skilled in the art. For example, the ratio of the active ingredients in the first section and the second section can be designed to be 1:1, and herein, for the specific amounts, 20mg each can be mixed on the basis of the active ingredient, esomeprazole. In the case of preparing as above, the durability of the efficacy can be superior, as compared to 40 mg drug product commercially available on the basis of the active ingredient.

### MODE FOR CARRYING OUT THE INVENTION

Hereinafter, the present invention will be explained through the examples. Meanwhile, it should be noted that the examples do not limit the scope of the present invention in any way.

### [Examples]

### 1. Elution test

As shown in the following Table 1, the first section pellet and the second section pellet, which are one embodiment according to the present invention, were prepared, and then the elution test was conducted.

The respective pellets were prepared as below.

### Device used - Glatt, Fluidized bed coater (GPCG-2)

Preparation of the first or second section drug layer - The drug layer was prepared by putting 70% ethanol in a stainless steel vessel, and adding the ingredients of the first or second section drug layer, except for sugar sphere, and stirring and completely dissolving it to prepare a coating solution of the drug layer, and then adding Sugar sphere into GPCG-2 and spraying the prepared coating solution.

Preparation of the first or second section separating layer - The protective layer was prepared by putting purified water in a stainless steel vessel and adding the ingredients of the first or second section separating layer and stirring and dispersing it to prepare a coating solution of the separating layer, and then putting the prepared drug layer pellet in GPCG-2 and spraying the prepared coating solution

Preparation of the first or second section enteric layer - The enteric layer was prepared by putting 95% ethanol in a stainless steel vessel and adding the ingredients of the first or second section enteric layer and stirring and dispersing it to prepare a coating solution of the enteric layer, and then putting the separating layer pellet comprising the prepared drug layer in GPCG-2 and spraying the prepared coating solution.

The elution test was conducted at pH 6.5, 900 mL, and 100 rpm of the first elution test method among the general test methods of the Korean Pharmacopoeia. The specific test conditions were as below.

### Devise used - Vankel, Dissolution system

### <Elution test device operation conditions>

Elution solution: pH 6.5 buffer solution_ 1L solution in which Potassium phosphate monobasic 6.805g and sodium hydroxide 1.164g were put in the container and purified water was added
Temperature: 37 ± 0.5 °C
Amount of elution solution: 900 mL
Rotation speed: 100 rpm

The conditions for this evaluation were set similar to *in-vivo* behavior of the drug, and it has been generally known that *in-vivo* behavior can be identified under the test conditions (*in-vitro*).

**[Table 1]**

| | | F1 | F2 |
|---|---|---|---|
| | Ingredient | first section | second section |
| Drug layer | Sugar sphere | 21.7 mg | 21.7 mg |
| | Esomeprazole Mg+ 2H₂O | 21.7 mg | 21.7 mg |
| | Hypromellose 2910 | 13.0 mg | 13.0 mg |
| | Polysorbate 80 | 1.8 mg | 1.8 mg |
| | Meglumine | | 65.1 mg |
| Separating layer | Hypromellose 2910 | 18.5 mg | 18.5 mg |
| | Talc | 18.5 mg | 18.5 mg |
| Enteric layer | Methacrylic Acid Copolymer Dispersi on | 12.8 mg | |
| | Methacrylic Acid Copolymer, Type B | | 45.3 mg |
| | Methacrylic Acid Copolymer, Type A | | 15.1 mg |
| | Talc | 6.6 mg | 17.8 mg |
| | Triethyl Citrate | 2.6 mg | 10.7 mg |

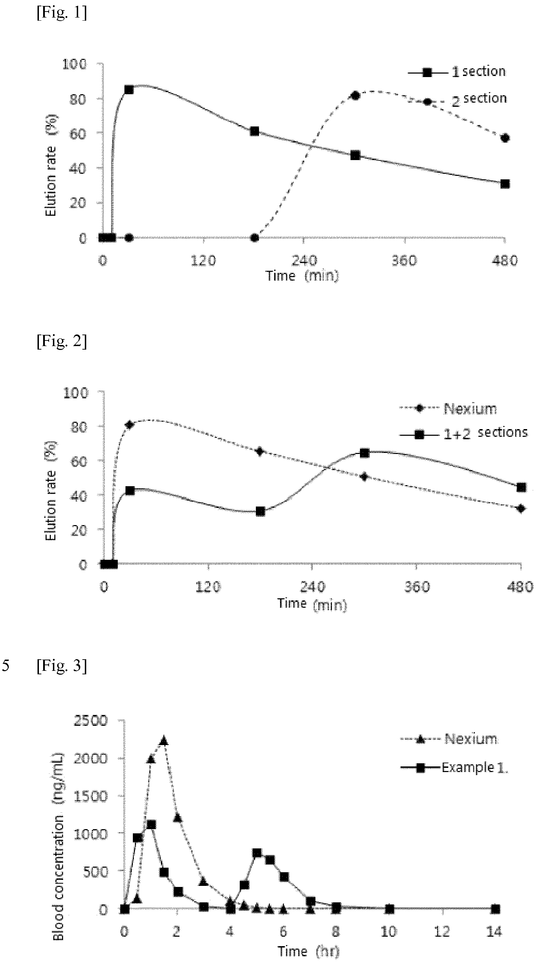

The result was the same as shown in Fig. 1.

As shown in Fig. 1, the first section pellet was released in the amount of 5% or less until 10 minutes after initiating the elution of esomeprazole and then the release reached up to 80% or more until 30 minutes, and the second section pellet was released in the amount of 5% or less until 3 hours after initiating the elution and then the release reached up to 70% or more until 5 hours. This is obvious when referring to the following Table 2. Table 2 shows the elution rates (%) at 10 min, 30 min, 180 min, 300 min, and 480 min points in Fig. 1.

**[Table 2]**

| Time (min) | First section | | Second section | |
|---|---|---|---|---|
| | Elution rate (%) | Standard deviation | Elution rate (%) | Standard deviation |
| 0 | 0.0 | 0.0 | 0.0 | 0.0 |
| 10 | 0.0 | 0.0 | 0.0 | 0.0 |
| 30 | 85.2 | 4.1 | 0.0 | 0.0 |
| 180 | 61.3 | 0.5 | 0.0 | 0.0 |
| 300 | 47.4 | 0.4 | 82.2 | 1.8 |
| 480 | 31.1 | 0.4 | 57.8 | 1.7 |

The background of the configuration of the first section and the second section lies in the residence time of the drug in the body. In the case of esomeprazole, it has been reported that as a drug remains in the gastrointestinal tract for a long time, the degradability of the drug is high. Thus, most of esomeprazole preparations minimize the degradation of the drug by fast releasing the drug in the gastrointestinal tract. However, said products have a drawback that they cannot prolong the duration time of the drug in the body. In order to improve this drawback, the present invention was configured such that in the small intestine, the first section induces the fast release of esomeprazole, and the second section induces the secondary release of esomeprazole at a specific time in consideration of the dissipation rate of the drug of the first section in the body for the prolongation of the duration time of the drug in the body.

### 2. Comparative elution test

The capsule was prepared by mixing F1 and F2 of Table 1 at a ratio of 1: 1 on the basis of the amount of the active ingredient and filling a soft capsule (Cap length 9.7-11.1 mm, Body length 16.5-18.5 mm), which is based on gelatin, with the assembly (F3) of the unit section comprising the composition of the following Table 3.

**[Table 3]**

| | Ingredient | F3 |
|---|---|---|
| Drug layer | Sugar sphere | 43.4 mg |
| | Esomeprazole Mg+ 2H₂O | 43.4 mg |
| | Hypromellose 2910 | 26.0 mg |
| | Polysorbate 80 | 3.6 mg |
| | Meglumine | 65.1 mg |
| Separating layer | Hypromellose 2910 | 37.0 mg |
| | Talc | 37.0 mg |
| Enteric layer | Methacrylic Acid Copolymer Dispersion | 12.8 mg |
| | Methacrylic Acid Copolymer, Type B | 45.3 mg |
| | Methacrylic Acid Copolymer, Type A | 15.1 mg |
| | Talc | 24.4 mg |
| | Triethyl Citrate | 13.3 mg |

The elution profiles of the capsule (esomeprazole 20 mg as the active ingredient of the first section, esomeprazole 20 mg as the active ingredient of the second section) which is one embodiment according to the present invention prepared with the compositions of the above Table 3 and of "Nexium Tab. (esomeprazole 40mg as the active ingredient)" which is commercially available drug product of esomeprazole were compared.

The result was the same as shown in Fig. 2.

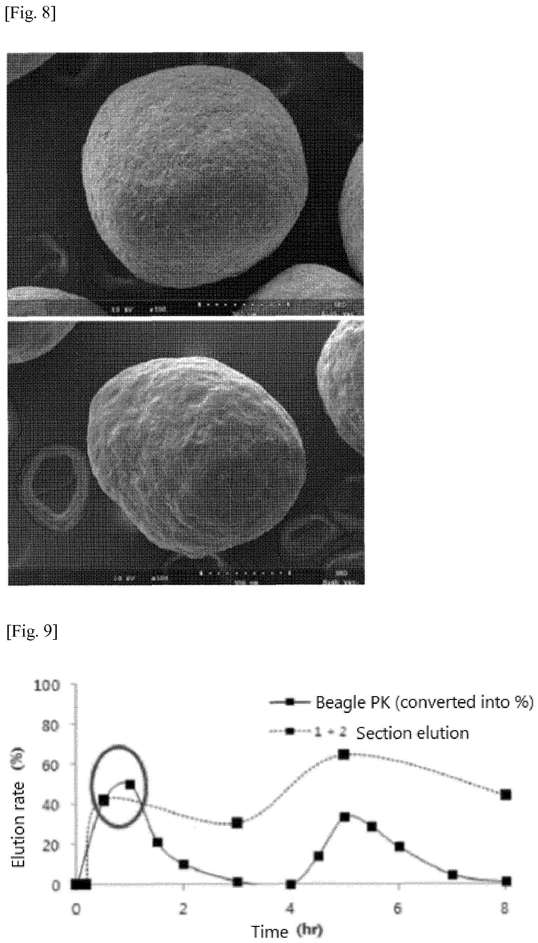

The evaluation of the subject elution is *in-vitro* elution file for predicting *in-vivo* behavior of the drug. That is, the presented elution conditions are to identify the matching with the *in-vivo* blood concentration profile of the drug (the time when the drug is released and the time when the drug is delayed, etc.) to identify whether the set elution test method was proper. Consequentially, the set elution test method confirmed the identity of the elution profile with the PK profile of the animal test as shown in Fig. 9 (30 minutes - 1 hour for which the drug of the first section is released *in-vivo* at the maximum - 1 hour the maximum blood concentration of the drug of the first section in the animal test / 5 hours for which the drug of the second section is released *in-vivo* at the maximum - 5 hours the maximum blood concentration of the drug of the second section in the animal test / 2-4.5 hours *in-vivo* delayed time of the drug between the first section and the second section and the reduction of the blood concentration of the drug of the first section and the second section in the animal test) so as to secure the propriety of the set elution test.

### 3. Animal test

Fistula was mounted on test animals, Beagle dogs, in which Hedenhain pouch was formed by the operation, and then test drug (F3) and control preparation (commercially available Nexium Tab 40 mg) were orally administered, and the gastric fluid and blood were collected after 0.5, 1, 1.5, 2, 3, 4, 4.5, 5, 5.5, 6, 7, 8, 10, and 14 hours and analyzed.

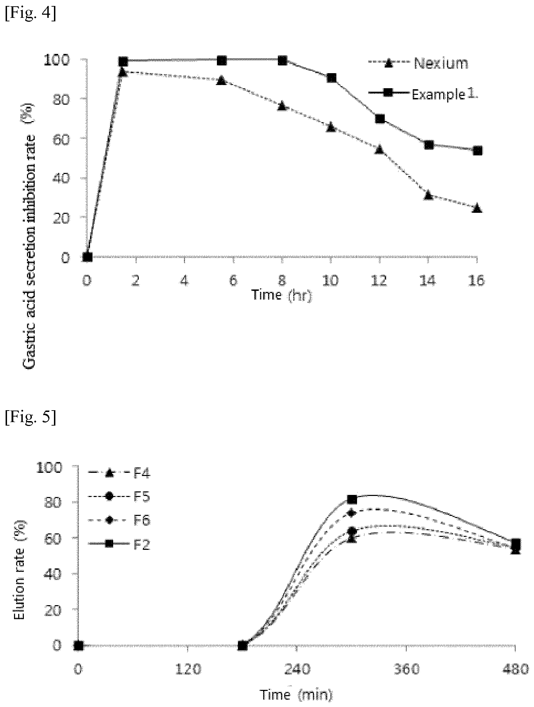

The results were the same as shown in Figs. 3 and 4.

The elution patterns of both the control preparation and the test drug showed significant correlation with the PK profile. However, in the pharmacodynamic aspect, the delayed-released test drug inhibited the gastric acid for longer time. Based on this result, it was confirmed that the test drug has the superior effect than the control preparation in the inhibition of Proton pump.

### 4. Solubilization effect test

In order to identify the effect of meglumine as the solubilizer of esomeprazole, the control test was conducted by changing the compositions as shown in the following Table 4 under the following conditions.

### Device used - Vankel, Dissolution system

### <Elution test device operation conditions>

Elution solution: pH 6.5 buffer solution_ 1L solution in which Potassium phosphate monobasic 6.805g and sodium hydroxide 1.164g were put in the container and purified water was added.
Temperature: 37 ± 0.5 °C
Amount of elution solution: 900 mL
Rotation speed: 100 rpm

**[Table 4]**

| | Ingredient | F4 | F5 | F6 | F2 |
|---|---|---|---|---|---|
| Drug layer | Sugar sphere | 21.7 mg | 21.7 mg | 21.7 mg | 21.7 mg |
| | Esomeprazole Mg+ 2H₂O | 21.7 mg | 21.7 mg | 21.7 mg | 21.7 mg |
| | Hypromellose 2910 | 13.0 mg | 13.0 mg | 13.0 mg | 13.0 mg |
| | Polysorbate 80 | 1.8 mg | 1.8 mg | 1.8 mg | 1.8 mg |
| | Meglumine | | 21.7 mg | 43.4 mg | 65.1 mg |
| Separating layer | Hypromellose 2910 | 18.5 mg | 18.5 mg | 18.5 mg | 18.5 mg |
| | Talc | 18.5 mg | 18.5 mg | 18.5 mg | 18.5 mg |
| Enteric layer | Methacrylic Acid Copolymer Dispersion | | | | |
| | Methacrylic Acid Copolymer, Type B | 31.7 mg | 36.3 mg | 40.8 mg | 45.3 mg |
| | Methacrylic Acid Copolymer, Type A | 10.6 mg | 12.1 mg | 13.6 mg | 15.1 mg |
| | Talc | 17.8 mg | 17.8 mg | 17.8 mg | 17.8 mg |
| | Triethyl Citrate | 10.7 mg | 10.7 mg | 10.7 mg | 10.7 mg |

The result was the same as shown in Fig. 5.

Solubilization of the drug was improved when meglumine was used.

### 5. Stability test

The stability of F3 which is one embodiment of the present invention was evaluated.

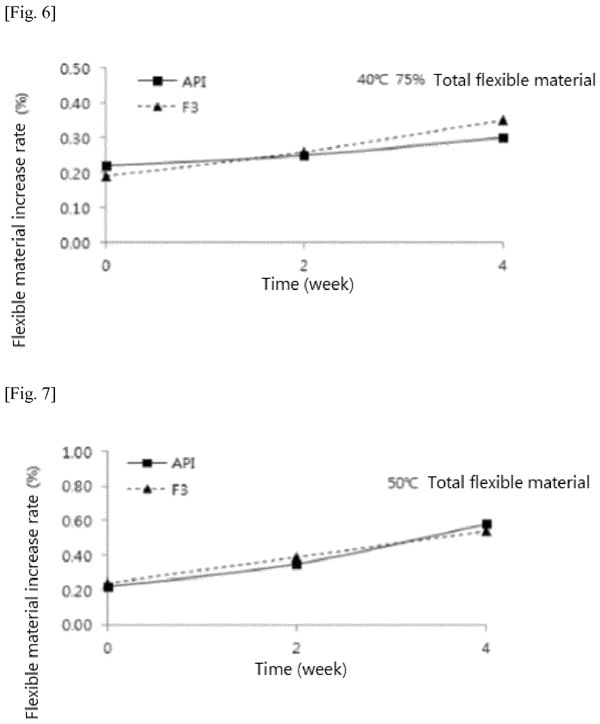

The total flexible materials were evaluated by conducting an Open severe test for the preparation (HDPE bottle with no cap at relative humidity 75% and 50°C). The flexible materials during each period were indicated with flexible material %. As the result, it was confirmed that the test drug of the present invention was stable since there was no significant difference between the raw material that has stability and the test drug (F3) as shown in Figs. 6 and 7.

### 6. Test for profile of pellet

The pellet was prepared with the compositions of the following Table 5 and then the profile was measured.

### Device used - Glatt, Fluidized bed coater (GPCG-2)

Preparation of the drug layer - The pellet was prepared by putting 70% ethanol in a stainless steel vessel, and adding the ingredients of the F2/F3 drug layer, except for sugar sphere, and stirring and completely dissolving it to prepare a coating solution of the drug layer, and then putting Sugar sphere into GPCG-2 and spraying the prepared coating solution to prepare the drug layer pellet, and then the profile was measured.

**[Table 5]**

| | Ingredient | F2 | F7 |
|---|---|---|---|
| Drug layer | Sugar sphere | 21.7 mg | 21.7 mg |
| | Esomeprazole Mg+ 2H₂O | 21.7 mg | 21.7 mg |
| | Hypromellose 2910 | 13.0 mg | 13.0 mg |
| | Polysorbate 80 | 1.8 mg | |
| | Monoglycerid | | 1.8 mg |
| | Meglumine | 65.1 mg | 21.7 mg |

The result was the same as shown in Fig. 8. This is the profile of the pellet in which only the drug layer was coated.

For constant drug release, the pellet needs to have the shape close to the spherical shape. In this test, the spherical shape was confirmed through the SEM (Scanning electron microscope) measurement after the preparation of the product using two kinds of surfactants whose hydrophile-lipophile balance (HLB) values are different, and as the result, it was confirmed that polysorbate having a higher HLB value (HLB ≥ 15) served as a preferable role in the formation of the spherical shape as compared to monoglyceride (HLB 3.3 ∼ 4.1) having a low HLB value.

## Claims

1. A preparation comprising a plurality of unit sections including a first section pellet comprising an inert core, a drug layer coated on the inert core, and an outermost layer coated with an enteric layer; and a second section pellet comprising an inert core, a drug layer coated on the inert core, and an outermost layer coated with an enteric layer, **characterized in that** the drug layer of the first section pellet contains esomeprazole as an active ingredient, and the drug layer of the second section pellet contains esomeprazole as an active ingredient and meglumine as a solubilizer, wherein in the first section, when testing with 100 rotations per minute according to the first elution test method, the active ingredient in the unit section is released in the amount of 5% or less relative to the total amount until 10 minutes after elution initiation, and the active ingredient is released in the amount of 80% or more relative to the total amount until 30 minutes, and in the second section, when testing with 100 rotations per minute according to the first elution test method, the active ingredient in the unit section is released in the amount of 5% or less relative to the total amount until 3 hours after elution initiation, and the active ingredient is released in the amount of 70% or more relative to the total amount until 5 hours.

2. The preparation according to claim 1, **characterized in that** the first section pellet further comprises a separating layer between the drug layer and the outermost layer coated with the enteric layer.

3. The preparation according to claim 1, **characterized in that** the second section pellet further comprises a separating layer between the drug layer and the outermost layer coated with the enteric layer, wherein the separating layer comprises a sustained release polymer.

4. The preparation according to claim 1, **characterized in that** the drug layer of the second section pellet further comprises a sustained release polymer.

5. The preparation according to claim 1, **characterized in that** the drug layer of the second section pellet further comprises a surfactant.

6. The preparation according to claim 5, **characterized in that** the surfactant is polysorbate.
